# EUROPEAN PATENT APPLICATION

(11) **EP 3 936 132 A1**
(43) Date of publication of application: **12.01.2022**
(21) Application number: 20184767.0
(22) Date of filing: 08.07.2020
(51) Int. Cl.: A61K 31/55, A61P 31/14

(54) **AZELASTINE AS ANTIVIRAL TREATMENT**

(71) Applicant: CEBINA GmbH, 1030 Vienna (AT)
(72) Inventor: NAGY, Eszter, 1070 Vienna (AT); NAGY, Gábor, 9400 Sopron (HU); SZIJÀRTO, Valeria, 1160 Vienna (AT); KONRAT, Robert, 1040 Vienna (AT)
(74) Representative: Loidl, Manuela Bettina

(57) **Abstract**

An Azelastine compound in an antiviral effective amount for use as an antiviral substance in a pharmaceutical preparation for use in prophylactic or therapeutic treatment of a subject in need of antiviral treatment.

## Description

### FIELD OF THE INVENTION

The present invention relates to novel uses of an anti-histamine drug compound for treating Coronaviridae virus infections, particularly methods and compounds for treating a SARS virus or MERS virus.

### BACKGROUND OF THE INVENTION

Coronaviruses are single-stranded RNA viruses, about 120 nanometers in diameter. They are susceptible to mutation and recombination and are therefore highly diverse. There are about 40 different varieties and they mainly infect human and non-human mammals and birds. They reside in bats and wild birds, and can spread to other animals and hence to humans.

There are four main subgroups (alpha, beta, gamma, and delta-coronavirus) based on their genomic structure. Alpha- and beta-coronaviruses infect only mammals, usually causing respiratory symptoms in humans and gastroenteritis in other animals. Until December of 2019, only six different coronaviruses were known to infect humans. Four of these (HCoV-NL63, HCoV-229E, HCoV-OC43 and HKU1) usually caused mild common cold-type symptoms in immunocompetent people and the other two have caused pandemics in the past two decades. In 2002-2003, the severe acute respiratory syndrome coronavirus (SARS-CoV) caused a SARS epidemic that resulted in a 10% mortality. Similarly, the Middle East respiratory syndrome coronavirus (MERS-CoV) caused a pandemic in 2012 with a 37% mortality rate.

In late 2019 and early 2020, a novel coronavirus, SARS-coronavirus 2 (SARS-CoV-2), which is closely related to SARS-CoV, was discovered to be the cause of a large and rapidly spreading outbreak of respiratory disease, including pneumonia. Since the novel coronavirus was recognized, the disease it caused was termed coronavirus disease 2019 (CoVID-19).

The SARS-related coronaviruses are covered by spike proteins that contain a variable receptor-binding domain (RBD). This RBD binds to angiotensin-converting enzyme-2 (ACE-2) receptor found in the heart, lungs, kidneys, and gastrointestinal tract, thus facilitating viral entry into target cells.

Azelastine, a phthalazine derivative, is an antihistamine available as an intranasal spray for the treatment of allergic and vasomotor rhinitis and as an ophthalmic solution for the treatment of allergic conjunctivitis. It is a racemic mixture, though there is no noted difference in pharmacologic activity between enantiomers, and was first granted FDA approval in 1996.

Gysi et al. ("Network Medicine Framework for Identifying Drug Repurposing Opportunities for COVID-19", arXiv:2004.07229 [q-bio.MN]) discloses a network-based toolset to COVID-19 to arrive at certain drug candidates based on their likely efficacy for COVID-19 patients, among them Azelastine, yet without any indication whether the antihistamine would have a direct impact on the virus underlying the disease.

### SUMMARY OF THE INVENTION

It is the objective of the present invention to provide new antiviral treatments, medicinal and pharmaceutical products which can be used to prevent from virus infection and/or virus spread, in particular in subjects that have been exposed to or infected with a virus, or who are at risk of being infected. The objective is solved by the subject of the present claims and as further described herein.

The invention provides for an Azelastine compound in an antiviral effective amount for use as an antiviral substance in a pharmaceutical preparation for use in prophylactic or therapeutic treatment of a subject in need of antiviral treatment.

Specifically, the Azelastine compound is Azelastine, or a pharmaceutically acceptable salt thereof, such as Azelastine hydrochloride.

According to a specific aspect, the pharmaceutical preparation is a medicinal product or a drug product. Specifically, the pharmaceutical preparation comprises the Azelastine compound and a pharmaceutically acceptable carrier.

Specifically, a disease condition is treated which is caused by or associated with an infection by a Coronaviridae virus.

According to a specific aspect, said virus is a Coronaviridae virus, preferably selected from the group consisting of a β-coronavirus, such as SARS-CoV-2, MERS-CoV, SARS-CoV-1, HCoV-OC43, or HCoV-HKU1, or an α-coronavirus, such as Humane Coronavirus NL63 (HCoV-NL63, New Haven coronavirus), HCoV-229E or Porcine epidemic diarrhea virus (PEDV).

The invention further provides for an Azelastine compound (as further described herein) in an antiviral effective amount for use as an antiviral substance in a pharmaceutical preparation for the prophylactic or therapeutic treatment of a disease condition caused by or associated with an infection by a virus which is a Coronaviridae virus, preferably selected from the group consisting of a β-coronavirus, such as SARS-CoV-2, MERS-CoV, SARS-CoV-1, HCoV-OC43, or HCoV-HKU1, or an α-coronavirus, such as HCoV-NL63, HCoV-229E or PEDV.

Specifically, the disease condition is common cold, infection of the nose, sinusitis, throat and larynx, bronchiolitis, diarrhea, rash on skin, or pneumonia, acute respiratory distress syndrome (ARDS). Specifically, a disease condition can be a symptom associated with any one or more of the foregoing. Specifically, a symptom to be treated can be any of coughing, sore throat, runny nose, sneezing, headache, and fever.

According to a specific aspect, the antiviral effective amount is effective in preventing infection of susceptible cells by the virus, thereby treating the disease condition. Specifically, susceptible cells are within or at a biological surface or a subject.

According to a specific aspect, the antiviral effective amount is 0.1 - 500 µg /dose, preferably below any one of 100, 90, 80, 70, 50, 50, 40, 30, 20, 10, 9, 8, 7, 6, 5, 4, 3, 2, 1.9, 1.8, 1.7, 1.6, 1.5, 1.4, 1.3, 1.2, 1.1 or 1 µg /dose. Specifically, the number of doses is up to 1 to 10 per day.

According to a specific aspect, said pharmaceutical preparation is formulated for local administration, such as for topical or topical mucosal administration, preferably for application to the upper and lower respiratory tract, nasal, pulmonary, intraoral, ocular, or dermal use, or for systemic administration, preferably by intravenous, intramuscular, subcutaneous, intradermal, transdermal, or oral administration. Typically, for parenteral administration, intravenous or peroral administration is preferred.

According to a specific aspect, said pharmaceutical preparation is administered to a subject as a spray, such as a nose spray, a powder, such as an instant powder or powder for inhalation, or by a healthcare device, such as e.g., comprising a surface or fabric impregnated with the Azelastine compound for inhalation, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

Specifically, antiviral formulations and administration forms are provided, such as for veterinary and for human use. Specifically, the formulations comprise a predetermined amount of the Azelastine compound as active ingredient; e.g., as a powder or granules; as a solution or a suspension in an aqueous or non-aqueous liquid; or as an oil-in-water liquid emulsion or a water-in-oil liquid emulsion.

According to a specific aspect, the Azelastine compound is used in an antiviral effective amount to provide a peak concentration (or maximum concentration) in blood or plasma, which is about 0.01-2 µg/mL, or up to any one of 1.6, 1.5, 1.4, 1.3, 1.2, 1.1, 1.0, 0.9, 0.8, 0.7, 0.6, 0.5, 0.4, 0.3, 0.2, or 0.1 µg/mL.

According to a specific aspect, the Azelastine compound is used in a formulation at a concentration of 1µM-10mM, preferably up to any one of 1 mM, 100 µM, 90 µM, 80 µM, 70 µM, 60 µM, or 50 µM. According to a specific embodiment, the concentration is 3-50 µM.

Specifically, a liquid solution or dispersion is used for nasal administration, such as by nose drops or a nasal spray, preferably wherein the antiviral effective amount is 1-1000 µg per nostril, preferably 1-500 µg per nostril, or up to any one of 200, 190, 180, 170, 160, 150, 140, 130, 120, 110, more preferably up to 100, 90, 80, 70, 60, 50, 40, 30, 20, 10, 5, 4, 3, 2 or 1 µg per nostril.

Specifically, a dose can be administered by nasal administration which is about 2-2000 µg per dose, preferably 2-1000 µg per dose, or up to any one of 400, 380, 360, 340, 320, 300, 280, 260, 240, 220, more preferably up to 200, 180, 160, 140, 120, 100, 80, 60, 40, 20, 10, 5, 4, 3, or 2 µg per dose.

A formulation is preferably applied as a nasal spray, nasal drops, an aerosol such as an aerosolized liquid or powder e.g., as a throat spray or for intrapulmonary administration.

Exemplary formulations may contain the Azelastine compound as an active ingredient in an amount of, for example, 0.001 to 2% (w/w), such as about 0.001%, 0.002%, 0.003%, 0.004%, 0.005%, 0.006%, 0.007%, 0.008%, 0.009%, 0.01%, 0.02%, 0.05%, 0.1%, 0.15%, 0.2%, 0.3%, 0.4%, 0.5%, 0.6%, 0.7%, 0.8%, 0.9%, 1.0%, 1.1 %,1.2%,1.3%,1.4%,1.5%,1.6%,1.7%, 1.8%, 1.9%, or 2.0% (w/w).

Specifically, a volume of 100 - 1000 µL per dose can be applied in a sprayable formulation, e.g., up to 500 µL spray volume. According to a specific example, a nasal spray may deliver a volume of about 100-150 µL per spray. Typically, two sprays are applied per nostril one or twice daily.

When using a spray, a metered spray is preferably used to administer a certain spray volume or dose per puff.

Formulations suitable for intrapulmonary administration may have a particle size in the range of 0.1 to 500 microns, which can be administered by inhalation through the nasal passage or by inhalation through the mouth so as to reach the alveolar sacs. Suitable formulations include aqueous or oily solutions of the Azelastine compound. Formulations suitable for aerosol or dry powder administration may be prepared according to conventional methods and may be delivered with other therapeutic agents such as compounds used in the treatment or prophylaxis of lung inflammation or lung diseases.

Specifically, a liquid solution or dispersion is used for parenteral administration, such as by infusions or injections, preferably wherein the antiviral effective amount provides a dose of about 1-500 mg.

Specifically, a single loading dose of about 1-500 mg may be administered parenterally, followed by maintenance doses of about 10 - 200 mg, or about 100 mg, or about 200 mg, e.g., by daily administration for 1-10 days, or until a certain clinical response has been achieved.

Specifically, a tablet, gel or lozenge is used for oral administration, preferably wherein the antiviral effective amount is 1 µg - 12 mg per dose, preferably up to any one of 5, 4, 3, 2, or 1 mg; or up to 100 µg per dose.

Specifically, a tablet comprising the Azelastine compound may be used which can be administered once to three times a day.

Formulations suitable for topical administration in the mouth include lozenges comprising the active ingredient in a flavored basis, such as sucrose, acacia or tragacanth, pastilles comprising the Azelastine compound in an inert basis such as gelatin and glycerin, sucrose, or acacia, or mouthwashes comprising the Azelastine compound in a suitable liquid carrier.

When formulated in a topically applied gel or ointment, the active ingredients may be employed with either a paraffinic or a water-miscible ointment base. Alternatively, active ingredients may be formulated in a cream with an oil-in-water cream base.

Formulations suitable for topical administration to the eye may include eye drops, gel or cream, wherein the Azelastine compound is dissolved or suspended in a suitable carrier, especially an aqueous solvent or oil/water emulsion.

According to specific examples, such formulations for a topical administration in the mouth, or a topically applied formulation such as a gel or ointment, may comprise the Azelastine compound in a concentration of, for example, 0.001 to 20% (w/w), such as about 0.001%, 0.002%, 0.003%, 0.004%, 0.005%, 0.006%, 0.007%, 0.008%, 0.009%, 0.01%, 0.02%, 0.05%, 0.1%, 0.15%, 0.2%, 0.3%, 0.4%, 0.5%, 0.6%, 0.7%, 0.8%, 0.9%, 1.0%, 2%, 3%, 4%, 5%, 6%, 7%, 8%, 9%, 10%, 11%, 12%, 13%, 14%, 15%, 16%, 17%, 18%, 9, or 20% (w/w).

According to a specific aspect, treatment with the Azelastine compound can be combined with a further treatment administering an Azelastine compound (which can be the same or a different compound) in another administration form. For example, treatment with an intranasal or throat spray comprising Azelastine Hydrochloride can be combined with a tablet comprising an Azelastine compound (which can be Azelastine Hydrochloride or different from Azelastine Hydrochloride).

According to a specific aspect, the Azelastine compound is administered as the sole antiviral substance, or wherein treatment is combined with a further treatment such as an additional antiviral, anti-inflammatory and/or antibiotic treatment e.g., comprising administration of one or more antiviral substances or agents, and/or one or more anti-inflammatory and/or antibiotic substances or agents, by one or more different preparations and/or one or more different routes of administration.

Specifically, the Azelastine compound can be combined with one or more additional active therapeutic agents in a unitary dosage form for simultaneous, co-administration or sequential administration to a subject. The combination therapy may e.g., be administered as a simultaneous, parallel or sequential regimen. When administered sequentially, the combination may be administered in two or more administrations.

According to a specific aspect, a subject is treated who has been infected or is at risk of being infected with said virus, preferably a human being, or a non-human mammal, such as a dog, cat, horse, camelids, cattle or pig.

Specifically, the subject is or has been exposed to a virus, or is otherwise at risk of being infected with the virus.

Specifically, the subject has been determined or diagnosed of being infected with the virus.

In specific embodiments, a subject is treated which is a diseased subject or patient suffering from Coronaviridae virus-caused disease, e.g., a SARS virus-caused disease, upon getting in contact with the pathogen, such as COVID19, or COVID19-associated pneumonia.

The invention further provides for the Azelastine compound as described herein for use as an antiviral substance in a medicinal product for treating a biological surface to prevent from virus infection and/or virus spread.

According to a specific aspect, said medicinal product is formulated for topical use, preferably for application to the upper and lower respiratory tract, nasal, pulmonary, intraoral, ocular, or dermal use.

Topical application typically refers to the surface of the skin, a wound, and/or mucosal cells or tissues (e.g., alveolar, buccal, lingual, masticatory, or nasal mucosa, etc.).

According to a specific aspect, said medicinal product is used in a formulation suitably used for topical administration, in particular mucosal administration, such as a spray, solution, dispersion, dry powder, or aerosolized liquid or powder.

According to a specific aspect, the Azelastine compound is applied to a biological surface in an antiviral effective amount, preferably wherein the amount is 1 ng -1000 ng per cm², preferably 10 - 800 ng / cm², or up to any one of 800, 700, 600, 500, 400, 300, 200, 100, 90, 80, 70, 60, 50 ng/ cm².

Any of the medicinal products suitably used for topical treatment as described herein can be used for treating the biological surface.

According to a specific aspect, the biological surface comprises or consists of a mucosal surface which is infected or at risk of being infected with said virus.

The invention further provides for the use of an Azelastine compound as described herein, as viral disinfectant, in particular suitable for treating a biological surface, or a non-biological surface, such as sanitary devices face masks, etc. Specifically, animate or non-animate surfaces can be treated using the disinfectant.

Specifically, the viral disinfectant is an antiviral preparation, such as a medicinal product.

According to specific aspect, the invention provides for an Azelastine compound as described herein, for use in preventing or treating a Coronaviridae viral infection, in particular a SARS virus infection, in a human or non-human mammal.

According to a specific aspect, a kit is provided which comprising one or more individual dosage units of the Azelastine compound as further described herein, and directions for their use in treating a Coronaviridae viral infection or a Coronaviridae virus-caused disease, in a human or non-human mammal.

According to a specific aspect, the invention provides for an antiviral pharmaceutical preparation comprising the Azelastine compound as further described herein and a pharmaceutically acceptable carrier.

Specifically, the pharmaceutical preparation is provided for medical use, in particular, for use in the prophylactic or therapeutic treatment of a disease condition caused by Coronaviridae virus such as COVID19.

According to a specific aspect, the invention further provides for methods of treating a subject being infected or at risk of being infected with a virus such as a Coronaviridae virus, comprising administering an antiviral effective amount of the Azelastine compound, and respective medicinal products or pharmaceutical preparations as further described herein.

According to a further specific aspect, the invention provides for an antiviral preparation of the Azelastine compound as described herein (such as a medicinal product, pharmaceutical preparation or disinfectant) and methods of producing such antiviral preparation comprising formulating an antiviral effective amount of the Azelastine compound with a pharmaceutically acceptable carrier to produce an antiviral preparation, in particular a medicinal product or pharmaceutical preparation.

The topical administration of any of the antiviral preparations as described herein (such as a medicinal product, pharmaceutical preparation or disinfectant) to a biological surface is preferably such that upon a certain contact time of e.g. 10 minutes to 24 hours, and/or up to 24, 18, 12, 6, 5, 4, 3, 2, or 1 hour(s), contacting results in at least a 0.5-fold (by half) reduction, or 1-log, 2-log, 3-log, 4-log, 5-log reduction in a virus on said surface.

### FIGURES

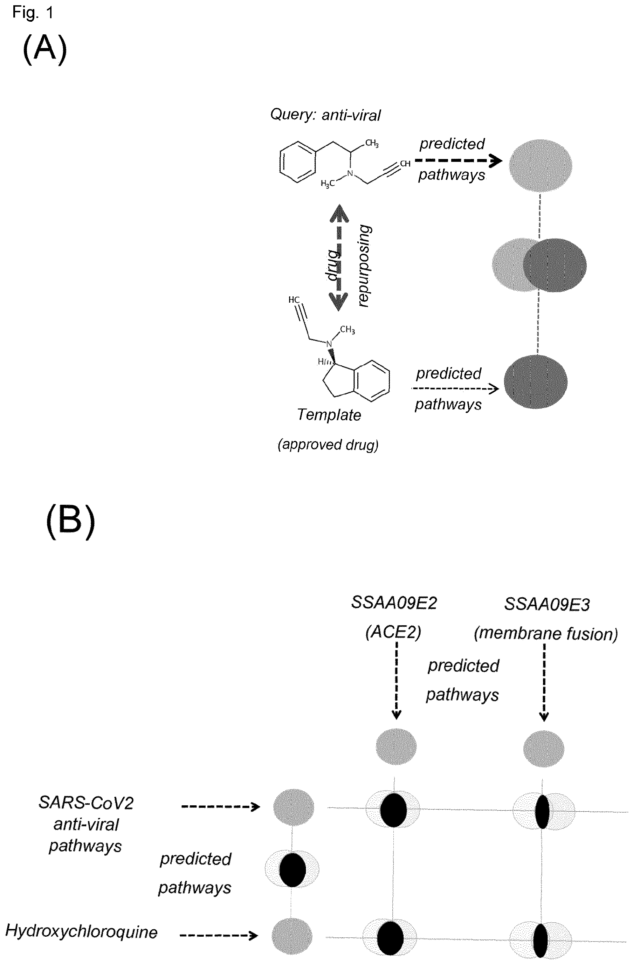

**Figure 1** shows the illustration of the Shannon entropy approach for the identification of drug homologs with matching pathway profiles (A) and the identification of SARS-CoV-2 relevant pathways (B). Pathway profiles were calculated for Hydroxychloroquine and SARS inhibitors with well-defined mechanisms and mode of actions: SSAA09E2, small molecule ACE2 inhibitor and SSAA09E3, a general inhibitor of virus host membrane fusion.

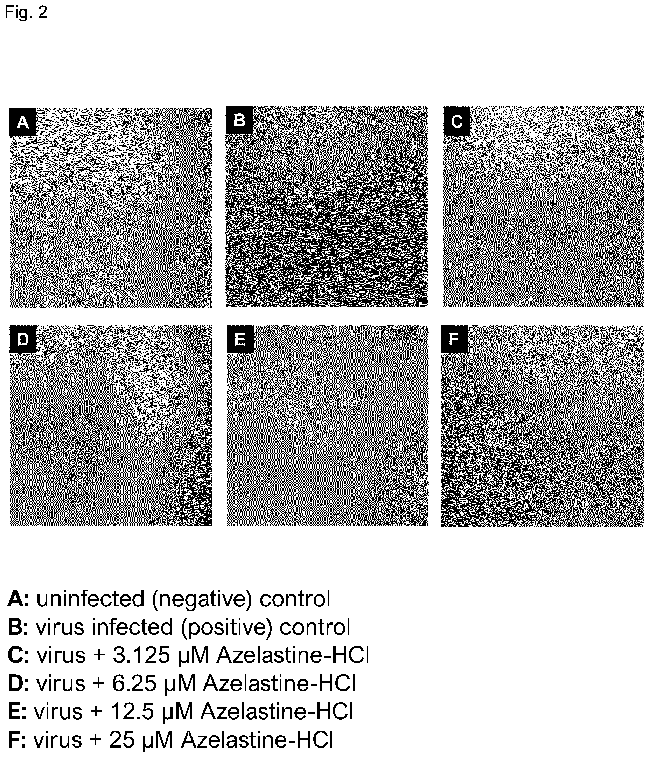

**Figure 2** shows the prevention of the cytopathic effect of SARS-CoV-2 caused to Vero E6 cells based on phasis contrast microscopic pictures. **A:** Uninfected (control) culture; **B:** Cells infected with SARS-CoV-2; **C-F:** Cells infected with SARS-CoV-2 in the presence of control buffer (containing 0.5% DMSO); **D-G:** Cells infected with SARS-CoV-2 in the presence of increasing concentrations of Azelastine-HCI: 3.125, 6.25, 12.5 and 25 µM. Cells were infected with MOI 0.1 SARS-Co-V2 virus for 30 min, then the culture medium was removed and replaced with fresh culture medium without virus (but containing Azelastine), and microscopic pictures taken 48 hours post infection.

### DETAILED DESCRIPTION OF THE INVENTION

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-10% or +/-5% of the given value.

The term "Azelastine compound" as used herein shall refer to Azelastine, or a salt thereof.

The molecular formula of Azelastine is C₂₂H₂₄ClN₃O with the following chemical structure (I):

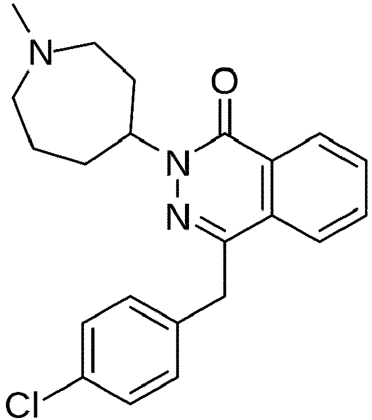

IUPAC name: 4-[(4-chlorophenyl)methyl]-2-(1 -methylazepan-4-yl)phthalazin-1 - one.
CAS number: 58581-89-8.

The Azelastine salt is preferably a pharmaceutically acceptable salt, such as for example a hydrochloride.

Azelastine hydrochloride is the hydrochloride salt form of azelastine, and used as an anti-histamine drug compound formulated as a metered-spray solution for intranasal administration. Commercial products comprising Azelastine hydrochloride are provided as nasal spray containing 0.1% or 0.15% Azelastine hydrochloride USP in an aqueous solution at pH 6.8 ± 0.3.

Azelastine hydrochloride occurs as a white or almost white, crystalline powder. It is sparingly soluble in water and soluble in ethanol and dichloromethane.

Molecular formula : C₂₂H₂₅Cl₂N₃O.

IUPAC name: 4-[(4-chlorophenyl)methyl]-2-(1 -methylazepan-4-yl)phthalazin-1 - one;hydrochloride.
CAS numbers: 58581-89-8; 37932-96-0; 79307-93-0.

The choice of an Azelastine salt is determined primarily by how acid or basic the chemical is (the pH), the safety of the ionized form, the intended use of the drug, how the drug is given (for example, by mouth, injection, or on the skin), and the type of dosage form (such as tablet, capsule, or liquid).

The term "pharmaceutically acceptable" also referred to as "pharmacologically acceptable" means compatible with the treatment of animals, in particular, humans. The term pharmacologically acceptable salt includes both pharmacologically acceptable acid addition salts and pharmacologically acceptable basic addition salts.

The term" pharmacologically acceptable acid addition salt" as used herein means any non-toxic organic or inorganic salt of any base compound of the disclosure, or any of its intermediates. Basic compounds of the disclosure that may form an acid addition salt include, for example, compounds that contain a basic nitrogen atom. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either the mono-, di- or the triacid salts can be formed, and such salts may exist in either a hydrated, solvated or substantially anhydrous form. In general, the acid addition salts of the compounds of the disclosure are more soluble in water and various hydrophilic organic solvents, and generally demonstrate higher melting points in comparison to their free base forms. The selection of the appropriate salt will be known to one skilled in the art. Other non-pharmacologically acceptable acid addition salts, e.g. oxalates, may be used, for example, in the isolation of the compounds of the disclosure, for laboratory use, or for subsequent conversion to a pharmacologically acceptable acid addition salt.

The term "pharmacologically acceptable basic salt" as used herein means any non-toxic organic or inorganic basic addition salt of any acid compound of the invention, or any of its intermediates, which are suitable for or compatible with the treatment of animals, in particular humans. Acidic compounds of the invention that may form a basic addition salt include, for example compounds that contain carboxylic acid, sulfonic acid, sulfinic acid, sulfonamide, N-unsubstituted tetrazole, phosphoric acid ester, or sulfuric acid ester. Illustrative inorganic bases which form suitable salts include lithium, sodium, potassium, calcium, magnesium, or barium hydroxide. Illustrative organic bases which form suitable salts include aliphatic, alicyclic or aromatic organic amines such as methylamine, trimethylamine and picoline or ammonia. The selection of the appropriate salt will be known to a person skilled in the art. Other non-pharmacologically acceptable basic addition salts, may be used, for example, in the isolation of the compounds of the invention, for laboratory use, or for subsequent conversion to a pharmacologically acceptable basic addition salt. The formation of a desired compound salt is achieved using standard techniques. For example, the neutral compound is treated with a base in a suitable solvent and the formed salt is isolated by filtration, extraction or any other suitable method.

The term "antiviral" as used herein shall refer to any substance, drug or preparation, that effects the biology of a virus and attenuates or inhibits viral attachment, entry, replication, shedding, latency or a combination thereof, resulting in reduction of viral load or infectivity. The terms "attenuating," "inhibiting," "reducing," or "prevention," or any variation of these terms, when used in the claims and/or the specification includes any measurable decrease or complete inhibition to achieve a desired result, e.g., reduction in the risk of viral infection (pre-exposure), or reduction of post-exposure viral survival, load, or growth.

Exemplary antiviral preparations described herein are medicinal products, pharmaceutical preparations, or disinfectants, for *in vivo, ex vivo* or *in vitro* use.

The term "biological surface" as used herein shall refer to a surface comprising viable cells, such as mammalian (human or non-human animal) cells, including e.g., a biological tissue surface, such as a surface or epithelial or dermal tissue (e.g. skin), mucosal tissue, or membrane tissue.

The term "effective amount" with respect to an antiviral effect as used herein, shall refer to an amount (in particular a predetermined amount) that has a proven antiviral effect. The amount is typically a quantity or activity sufficient to, when applied to a surface or administered to a subject effect beneficial of desired results, including antiviral or clinical results, and, as such, an effective amount or synonym thereof depends upon the context in which it is being applied.

An effective amount of a pharmaceutical preparation or drug is intended to mean that amount of a compound that is sufficient to treat, prevent or inhibit a disease, disease condition or disorder. Such an effective dose specifically refers to that amount of the compound sufficient to result in healing, prevention or amelioration of conditions related to diseases or disorders described herein.

In the context of disease, effective amounts (in particular prophylactically or therapeutically effective amounts) of an Azelastine compound as described herein are specifically used to treat, modulate, attenuate, reverse, or affect a disease or condition that benefits from its antiviral effect. The amount of the compound that will correspond to such an effective amount will vary depending on various factors, such as the given drug or compound, the formulation, the route of administration, the type of disease or disorder, the identity of the subject or host being treated, the assessment of the medical situations and other relevant factors, but can nevertheless be routinely determined by one skilled in the art.

A treatment or prevention regime of a subject with an effective amount of the Azelastine compound described herein may consist of a single application or administration, or alternatively comprise a series of applications and administrations, respectively. For example, the Azelastine compound may be used at least once a month, or at least once a week, or at least once a day. However, in certain cases of an acute phase, e.g. upon suspected or confirmed exposure to a virus, or after virus infection has been determined, the Azelastine compound may be used more frequently e.g., 1-10 times a day.

Specifically, a combination therapy is provided which includes treatment with the preparation described herein and standard therapy of a Coronaviridae virus-caused disease.

Doses may be applied in combination with other active agents such as antiviral agents, anti-inflammatory drugs or antibiotics, e.g. upon the subject's risk of viral spread, so to prevent a pathogen associated reaction.

Treatment can be combined with an antiviral, anti-inflammatory or antibiotic treatment, preferably wherein a pharmaceutical preparation is administered before, during (e.g., by co-administration or in parallel), or after said antiviral, anti-inflammatory or antibiotic treatment.

Specifically, the Azelastine compound described herein can be combined with an additional antiviral agent, which can be an Azelastine compound, e.g. the same of a different compound. Specific embodiments refer to further antiviral agents selected from an ACE2 inhibitor, a viral protein M2 ion channel inhibitor, a neuraminidase inhibitor, an RNA replication and translation inhibitor and a polymerase inhibitor. The antiviral agent may be amantadine or rimantadine. Specifically, the antiviral agent may be oseltamivir, zanamivir, peramivir, ribavirin, lopinavir, or ritonavir. Specific further antiviral examples are those suitably used for biological surface treatment such as carrageenan, or those currently under investigation for treating SARS-Cov2 infections, such as hydroxychloroquine, or remdesivir.

Specifically, the Azelastine compound is combined with an anti-inflammatory agent such as standard steroidal anti-inflammatory drugs, glucocorticoids and nonsteroidal anti-inflammatory drugs (NSAID's). Suitable NSAID's include, but are not limited to ibuprofen, naproxen, fenoprofen, ketoprofen, flurbiprofen, oxaprozin, indomethacin, sulindac, etodolac, ketorolac, diclofenac, nabumetone, piroxicam, meloxicam, tenoxicam, droxicam, lornoxicam, isoxicam, mefenamic acid, meclofenamic acid, flufenamic acid, tolfenamic acid and celecoxib. Suitable steroidal anti-inflammatory agents include, but are not limited to, corticosteroids such as synthetic glucocorticoids. Specific examples are fluticasone, COX-2 inhibitors, ibuprofen, hydroxychloroquine, heparin, LMW heparin, hirudine, or immunosuppressants, such as azathioprine, cyclosporin A, or cyclophosphamide.

Specifically, the Azelastine compound is combined with an antibiotic such as a beta lactam antibiotic, an aminoglycoside antibiotic, an ansamycin, a carbacephem, a carbapenem, a cephalosporin, a glycopeptide, a lincosamide, a lipopeptide, a macrolide, a monobactam, a nitrofuran, an oxazolidinone, a polypeptide, a sulfonamide, Clofazimine, Dapsone, Capreomycin, Cycloserine, Ethambutol, Ethionamide, Isoniazid, Pyrazinamide, Rifampicin, Rifabutin, Rifapentine, Streptomycin, Arsphenamine, Chloramphenicol, Fosfomycin, Mupirocin, Platensimycin, Quinupristin/Dalfopristin, Thiamphenicol, Tigecycline, Tinidazole, Trimethoprim, Teixobactin, Malacidins, Halicin, clindamycin, vancomycin, metronidazole, fusidic acid, thiopeptides, fidaxomicin, quinolons, tetracyclins, omadacycline, rifamycin, kibdelomycin, oxazolidinone, ketolides, thiazolides, amixicile, teicoplanin, ramoplanin, oritavancin, lantibiotics, capuramycin, surotomycin, thuricin, endolysin, avidocin CD, cadazolid, ramizol, defensins, ridinilazole, medium-chain fatty acids, phages, berberine, lactoferrin.

Specifically, treatment with the Azelastine compound described herein can be combined with a treatment administering at least one other therapeutic agent selected from the group consisting of a corticosteroid, an anti-inflammatory signal transduction modulator, a 2-adrenoreceptor agonist bronchodilator, an anticholinergic, a mucolytic agent, hypertonic saline and other drugs for treating a Coronaviridae virus infections; or mixtures thereof. Specific pharmaceutical compositions may particularly include one or more anti-inflammatory agents, and/or analgesics, PPAR-γ agonists and immune response modulators.

The length of the treatment period depends on a variety of factors, such as the severity of the disease, either acute or chronic disease, the age of the patient, and the concentration of the Azelastine compound. It will also be appreciated that the effective dosage used for the treatment or prophylaxis may increase or decrease over the course of a particular treatment or prophylaxis regime. Changes in dosage may result and become apparent by standard diagnostic assays known in the art.

According to a specific aspect, a medicinal product or pharmaceutical composition described herein contains an effective amount of the Azelastine compound as defined herein. The preparation described herein may be provided for single or multiple dosage use.

Unit-dose or multi-dose containers may be used, for example, sealed ampoules and vials, or multi-use sprays, and may be stored comprising a liquid or dry phase, e.g., in a freeze-dried (lyophilized) condition requiring only the addition of the sterile liquid carrier, for example water for injection, immediately prior to use. Preferred unit dosage formulations are those containing a daily dose or unit daily sub-dose, or multiple doses, of the Azelastine compound.

The term "single-dose" as used herein is understood in the following way. A single-dose or amount for single-use is the amount intended for administration that is meant for use in a single subject, such as a patient, either human or animal for a single case/procedure/administration. Packages comprising the single-dose are typically labelled as such by the manufacturer. The single-dose amount is specifically understood as a daily dose for an individual, like a child or adult, to provide an effective amount.

The medicinal product or pharmaceutical composition described herein is specifically provided as human or veterinary medicinal product or pharmaceutical composition. Medicinal products are understood as substances that are used to treat diseases, to relieve complaints, or to prevent such diseases or complaints in the first place. This definition applies regardless of whether the medicinal product is administered to humans or to animals. The substances can act both within or on the body.

The medicinal product or pharmaceutical composition described herein preferably contains one or more pharmaceutically acceptable auxiliaries and is in a pharmaceutical form which allows the active pharmaceutical compound to be administered with high bioavailability. Suitable auxiliaries may be, for example, based on cyclodextrins. Suitable formulations might for example incorporate synthetic polymeric nanoparticles formed of a polymer selected from the group consisting of acrylates, methacrylates, cyanoacrylates, acrylamides, polylactates, polyglycolates, polyanhydrates, polyorthoesters, gelatin, albumin, polystyrenes, polyvinyls, polyacrolein, polyglutaraldehyde and derivatives, copolymers and mixtures thereof.

Specific medicinal products or pharmaceutical compositions described herein comprise the Azelastine compound and a pharmaceutically acceptable carrier or excipient. A "pharmaceutically acceptable carrier" refers to an ingredient in a formulation for medicinal or medical use, other than an active ingredient, which is nontoxic to a subject. A pharmaceutically acceptable carrier includes, but is not limited to, a buffer, excipient, stabilizer, or preservative, and in particular saline, phosphate buffered saline, dextrose, glycerol, ethanol, and the like.

The Azelastine compound as used herein can be formulated with conventional carriers and excipients, which will be selected in accord with ordinary practice.

Pharmaceutically acceptable carriers generally include any and all suitable solvents, dispersion media, coatings, antiviral, antibacterial and antifungal agents, isotonic and absorption delaying agents, and the like that are physiologically compatible with an antiviral small molecule compound or related composition or combination preparation described herein.

According to a specific aspect, the Azelastine compound can be combined with one or more carriers appropriate a desired route of administration. The Azelastine compound may be *e.g*., admixed with any of lactose, sucrose, starch, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, polyvinyl alcohol, and optionally further tableted or encapsulated for conventional administration. Alternatively, the Azelastine compound may be dispersed or dissolved in saline, water, polyethylene glycol, propylene glycol, carboxymethyl cellulose colloidal solutions, ethanol, corn oil, peanut oil, cotton seed oil, sesame oil, tragacanth gum, and/or various buffers. Other carriers, adjuvants, and modes of administration are well known in the pharmaceutical arts. A carrier may include a controlled release material or time delay material, such as glyceryl monostearate or glyceryl distearate alone or with a wax, or other materials well-known in the art.

Compounds as described herein may be provided in controlled release pharmaceutical ("controlled release formulations") in which the release of the Azelastine compound is controlled and regulated to allow less frequency dosing or to improve the pharmacokinetic or toxicity profile of a given active ingredient.

Pharmaceutical compositions may also be coated by conventional methods, typically with pH or time-dependent coatings, such that the subject agent is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, waxes, and shellac.

Additional pharmaceutically acceptable carriers are known in the art and described in, e.g., Remington: The Science and Practice of Pharmacy, 22nd revised edition (Allen Jr, LV, ed., Pharmaceutical Press, 2012). Liquid formulations can be solutions, emulsions or suspensions and can include excipients such as suspending agents, solubilizers, surfactants, preservatives, and chelating agents.

The preferred preparation is in a ready-to-use, storage stable form, with a shelf-life of at least one or two years.

The term "formulation" as used herein refers to a preparation ready-to-use in a specific way. Specifically, compositions described herein comprises the Azelastine compound, and a pharmaceutically acceptable diluent, carrier or excipient.

According to a specific aspect, formulations are provided comprising pharmaceutically acceptable vehicles for nasal, intrapulmonary, oral, topical, mucosal or parenteral administration. Administration may also be intradermal or transdermal. Also, the present disclosure includes such compounds, which have been lyophilized and which may be reconstituted to form pharmaceutically acceptable formulations for administration.

Specific medicinal products or pharmaceutical compositions described herein are formulated for intranasal administration or by another topical route e.g., onto biological surfaces, including e.g., mucosa or skin. Pharmaceutical carriers suitable for facilitating such means of administration are well known in the art.

Specifically, a nasal spray may be used containing 0.001% or 0.15% (w/w) Azelastine compound in an aqueous solution at pH 6.8 ± 0.3, optionally further containing any one or more of citric acid monohydrate, disodium hydrogen phosphate dodecahydrate, edetate disodium, hypromellose, purified water, sodium chloride, and a preservative such as benzalkonium chloride.

To administer the Azelastine compound by any route other than parenteral administration, it may be necessary to coat the active agent with, or co-administer the active agent with, a material to prevent its inactivation. For example, an appropriate carrier may be used, for example, liposomes, or a diluent. Pharmaceutically acceptable diluents include saline and aqueous buffer solutions.

The Azelastine compound can be orally administered, for example, with an inert diluent or an assimilable or edible carrier. For example, a preparation may be enclosed in a hard- or soft-shell gelatin capsule, or compressed into tablets. For oral therapeutic administration, the Azelastine compound may be incorporated with excipients and used in the form of ingestible tablets, buccal tablets, troches, capsules, elixirs, suspensions, syrups, wafers, and the like. The percentage of the compound in the compositions and preparations may, of course, be varied. The amount of the Azelastine compound in such therapeutically useful compositions is such that a suitable dosage will be obtained.

Tablets will contain excipients, glidants, fillers, binders, disintegrants, lubricants, flavors and the like. Granules may be produced using isomaltose. It is furthermore preferred to provide for a preparation formulated to act at the site of the mucosa, e.g. at mucosal sites (such as nose, mouth, eyes, esophagus, throat, lung), e.g. locally without systemic action. Aqueous formulations are prepared in sterile form, and when intended for delivery by other than oral administration generally will be isotonic.

The term "mucosal" with respect to administration or application or else mucosal use of a preparation for treating a subject or a respective formulation, refers to administration via the mucosal route, including systemic or local administration, wherein an active ingredient is taken up by contact with mucosal surfaces. This includes nasal, pulmonary, oral, or peroral administration and formulations, e.g. liquid, syrup, lozenge, tablet, spray, powder, instant powder, granules, capsules, cream, gel, drops, suspension, or emulsion.

Peroral formulations may include liquid solutions, emulsions, suspensions, and the like. The pharmaceutically acceptable vehicles suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, tragacanth, and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Other compositions useful for attaining systemic delivery of the Azelastine compound or respective preparations include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose, or glidants, lubricants, sweeteners, colorants, antioxidants and flavoring agents.

The Azelastine compound or respective preparations can also be administered topically to a subject, e.g., by the direct laying on or spreading of a composition containing same on the epidermal or epithelial tissue of the subject, or transdermally via a "patch". Such compositions include, for example, lotions, creams, solutions, gels and solids. These topical compositions may comprise an effective amount, usually at least about 0.001 wt %, or even from about 0.1 wt% to 5 wt%, or 1 wt % to about 5 wt %, of the Azelastine compound. Suitable carriers for topical administration typically remain in place on the skin as a continuous film, and resist being removed by perspiration or immersion in water. Generally, the carrier is organic in nature and capable of having dispersed or dissolved therein the therapeutic agent. The carrier may include pharmaceutically acceptable emollients, emulsifiers, thickening agents, solvents and the like.

Pharmaceutical compositions suitable for injectable use include sterile aqueous solutions (in particular where the compounds or pharmaceutically acceptable salts are water soluble) or dispersions and sterile powders for the extemporaneous preparation of sterile injectable solutions or dispersion. In particular, the composition is specifically sterile and fluid to the extent that easy syringability exists; it is stable under the conditions of manufacture and storage and preserved against the contaminating action of microorganisms such as bacteria and fungi.

Suitable pharmaceutically acceptable vehicles include, without limitation, any non-immunogenic pharmaceutical adjuvants suitable for oral, parenteral, nasal, mucosal, transdermal, intravascular (IV), intraarterial (IA), intramuscular (IM), and subcutaneous (SC) administration routes, such as phosphate buffer saline (PBS).

The term "subject" as used herein shall refer to a warm-blooded mammalian, particularly a human being or a non-human animal, including e.g., dogs, cats, rabbits, horses, cattle, and pigs. In particular the treatment and medical use described herein applies to a subject in need of prophylaxis or therapy of a disease condition associated with a Coronaviridae virus infection. Specifically, the treatment may be by interfering with the pathogenesis of a disease condition where a Coronaviridae virus is a causal agent of the condition. The subject may be a patient at risk of such disease condition or suffering from disease.

The term "at risk of" a certain disease conditions, refers to a subject that potentially develops such a disease condition, e.g. by a certain predisposition, exposure to virus or virus-infected subjects, or that already suffers from such a disease condition at various stages, particularly associated with other causative disease conditions or else conditions or complications following as a consequence of viral infection. The risk determination is particularly important in a subject, where a disease has not yet been diagnosed. This risk determination therefore includes early diagnosis to enable prophylactic therapy. Specifically, the Azelastine compound is used in subjects with a high risk, e.g. a high probability of developing disease.

The term "patient" includes human and other mammalian subjects that receive either prophylactic or therapeutic treatment. The term "patient" as used herein always includes healthy subjects. The term "treatment" is thus meant to include both prophylactic and therapeutic treatment.

Specifically, the term "prophylaxis" refers to preventive measures which is intended to encompass prevention of the onset of pathogenesis or prophylactic measures to reduce the risk of pathogenesis.

The term "therapy" as used herein with respect to treating subjects refers to medical management of a subject with the intent to cure, ameliorate, stabilize, reduce the incidence or prevent a disease, pathological condition, or disorder, which individually or together are understood as "disease condition". The term includes active treatment, directed specifically toward the improvement of a disease condition, prophylaxis directed specifically toward the prevention of a disease condition, and also includes causal treatment directed toward removal of the cause of the associated disease condition. In addition, this term includes palliative treatment designed for the relief of symptoms rather than the curing of the disease condition, and further curing a disease condition directed to minimizing or partially or completely inhibiting the development of the associated disease condition, and supportive treatment employed to supplement another specific therapy directed toward the improvement of the associated disease condition.

The foregoing description will be more fully understood with reference to the following examples. Such examples are, however, merely representative of methods of practicing one or more embodiments of the present invention and should not be read as limiting the scope of invention.

### EXAMPLES

### Example 1: Antiviral drug identification

The development of mathematical representations for small molecules (drugs) is a research area of immense value for modern pharmaceutical research and thus numerous molecular descriptors have been developed and exploiting 2D and/or 3D features of chemical structures. These descriptors have been very valuable in assessing quantitative structure-activity relationships. Specifically, atom-centered feature pairs have turned out to be of great relevance for drug discovery programs as they provide cost-effective approaches to high-throughput structure-activity relationship analysis, compound selection, virtual chemical screening and pharmacological profiling. One of the commonly used chemical descriptors is referred to as SHED, for Shannon Entropy Descriptor (1). In this approach the topological distributions of atom-centered feature pairs are quantified based on information theory (Shannon entropy). The particular benefit is that chemically different but topologically related chemical scaffolds can be identified using the SHED approach.

In order to identify new anti-viral drugs, a drug identification strategy was used based on a biochemical pathway-based intervention strategy. Clinically approved drugs that match a predefined mechanistic profile (mode-of-action) were identified as candidate anti-viral drugs using a Shannon entropy-based description of the inherent chemical features of small molecules (drugs). The rationale behind this approach is that ligands with similar Shannon entropy vectors will bind to similar protein targets.

Here the DRUGBANK database (2) was used, a repository of approved drugs and their experimentally verified protein targets. Drug similarities were assessed by calculating Euclidean distances (0.25 was taken as a cutoff value).

As a starting point for the analysis, available mechanistic information on SARS-CoV-2 infection was extracted from recent bioinformatics analysis (3) together with analysis of certain query anti-viral compounds. The pathway profiles of the query anti-viral compounds with known SARS-CoV2 activity (hydroxychloroquine (RS)-2-[{4-[(7-Chlor-4-chinolinyl)amino]pentyl}(ethyl)amino]ethanol; SSAA09E2 {N-[[4-(4-methylpiperazin-1-yl)phenyl]methyl]-1,2-oxazole-5-carboxamide}; and SSAA09E3 [N-(9,10-dioxo-9,10-dihydroanthracen-2-yl)benzamide]) were predicted using the Shannon entropy approach, and interestingly, showed significant mutual overlap.

Secondly, the pathway profiles for SARS inhibitors were predicted with well-defined mechanisms and mode of actions: SSAA09E2, a small molecule ACE2 inhibitor, and SSAA09E3, a general inhibitor of virus host membrane fusion (4). It was found that both inhibitors shared a considerable number of pathways with hydroxychloroquine. Summing up, the analysis showed that similar pathways are involved in SARS-CoV-2 infection, targeted by hydroxychloroquine and addressed by inhibitors with well-defined mechanisms. It was thus concluded that these pathways are highly relevant for antiviral activity and can serve as the basis for a novel drug repurposing for anti-viral activity by looking for clinically approved drugs that previously were not known to have anti-viral activity with matching pathway profiles.

Clinically approved drugs were explored for matching the individual pathway profiles. The predicted pathway profiles obtained for the different query compounds

(hydroxychloroquine, SSAA09E2 and SSAA09E3) were employed to screen the SELLECKCHEM database of approved (and commercially available) drugs. The rationale for candidate selection was based on simultaneous appearance of approved drugs in the different (predicted) datasets. Hydroxychloroquine and SSAA09E2 (ACE2 inhibitor) showed significant overlap among each other and with drugs obtained with the SARS-CoV-2 pathway profile. After eliminating drugs based on chemical composition, two approved drugs were selected as candidate anti-virals for further testing in an in vitro SARS-CoV-2 infection model: Azelastine and Maraviroc.

References:
(1) Gregori-Puigjane, E. and Mestres, J. SHED: Shannon Entropy Descriptors from Topological Feature Distributions. J.Chem.Inf.Model. 46, 1615-1622 (2006)
(2) Wishart, DS., Knox, C., Guo, AC:, Cheng, D., Shrivastava, S., Tzur, D., Gautam, B. and Hassanali, M. DrugBank: a knowledgebase for drugs, drug action and drug targets. Nucleic Acids Res. D901-D906, (2008)
(3) Zhou, Y, Hou, Y., Shen, J, Huang, Y., Martin, W. and Cheng, F. Network-based drug repurposing for novel coronavirus 2019-nCoV/SARS-CoV-2. Cell Discovery 6,14-32, (2020)
(4) Adedeji, AO., Severson, W., Jonsson, C., Singh, K., Weiss SR. and Sarafanos, SG. Novel Inhibitors of Severe Acute Respiratory Syndrome Coronavirus Entry That Act by Three Distinct Mechanisms. J Virol. 87, 8017-8028, (2013)

### Example 2: Preventing virus infection of cells by SARS-CoV-2

To detect the effect of Azelastine-HCI on SARS-CoV-2 infection, the ACE2 expressing Vero E6 (monkey kidney) cells were infected with SARS-CoV-2 in the absence or presence of Azelastine-HCI and the cytopathogenic effect was evaluated by microscopic examination of the cells.

### Experimental procedure:

Vero E6 cells (ATCC CRL-1586) were seeded on 96-well plates. After 2 days the cell cultures reached confluency and formed a homogenous monolayer. The cells were fed with fresh cell culture medium (DMEM+ FBS?). Azelastine-HCl (Seleckchem cat# S2552, 10mM stock solution dissolved in DMSO) and Maraviroc (Seleckchem cat# S2003, 10mM stock solution dissolved in DMSO), an anti-HIV antiviral agent, were added to the cell culture medium at 50, 25, 12.5, 6.25 and 3.125 µM final concentrations (dilutions were prepared in culture medium). For viral infection the SARS-CoV-2 virus was added to the supernatant at MOI 0.1 (multiplicity of infection: 1 viral particle to 10 cells) immediately after the culture medium exchange (basically simultaneously). The virus stock was prepared by propagation in Vero E6 cells and the infectious titre determined. After 30 min incubation with the virus, the culture medium was removed and replaced with fresh culture medium containing Azelastine-HCl or Maraviroc at the above concentrations (co-administration: simulating prevention). The experiments were also performed with Azelastine in a way when the drug was given only after the 30 min incubation with the virus, but not during this period (post-infection administration: post-exposure/therapeutic setting). 48 hours post infection the cells were evaluated by microscopic observation and then the supernatants were collected and stored at -80 °C for quantitative PCR analysis. Viral RNA was extracted from the culture supernatant samples with the Monarch Total RNA Miniprep Kit (New England BioLabs, Cat#: T2010S) according to the manufacturer's instructions (https://international.neb.com/- /media/nebus/files/manuals/manualt2010.pdf?rev=183dcb38a7624a53abdf6ad0316afd 4f&hash=AD8D5F25CDBD724448B8065FOFOCC959). Briefly, 300µl Lysis buffer was mixed with 100µl culture supernatant, the gDNA contamination was removed with the dedicated column (retaining DNA), and the flow-through containing RNA was applied to the RNA-binding column. After washing the column, the RNA was eluted with H₂O, and samples stored at -80 °C till analysis. After the reverse transcription reaction, DNA was amplified with the F, R and P2 primers from the RdRp gene (https:llwww.eurosurveillance.org/content/table/10.2807/1560-7917.ES.2020.25.3.2000045.t1?fmt=ahah&fullscreen=true) using the droplet PCR kit (BioRad ddPCR^{™}, https://www.bio-rad.com/de-at/applications-technologies/droplet-digital-pcr-ddpcr-technology?ID=MDV31M4VY).

The results of the RT-PCR reaction were quantified and calculated as viral particle/µl.

### Results:

The confluent, homogenous layer of cells (uninfected, Fig. 2A) is disrupted and "holes" appear indicating cell death due to the virus (Fig. 2B). In the presence of Azelastine-HCI at all tested concentrations, the SARS-CoV-2 infected cells were significantly protected from dying providing evidence of direct anti-viral effect (Fig. 2C-F). Maraviroc was not effective at all at lower concentrations, and even the higher concentrations (12.5 to 50 µM) had only marginal protective effect with high cytopathogenic scores (Table 1). Surprisingly, Azelastine turned out to be an effective antiviral substance, which is at least as effective as hydroxychloroquine.

These data suggest that an Azelastine compound was able to stop infection by SARS-CoV-2 immediately as soon as it was applied to the cells. Since the virus needs to enter cells to multiple and spread into the body, Azelastine is expected to prevent COVID-19 right at the place where the virus infects the human body, on the mucosal surfaces of the respiratory tract.

**Table 1**

| **Compound** | **50 µM** | **25 µM** | **12.5 µM** | **6.25 µM** | **3.125 µM** |
|---|---|---|---|---|---|
| Azelastine-HCl | nd | 0 or 1 | 0 or 1 | 2 | 2 |
| Maraviroc | 3 | 3 | 3 | 4 | 4 |

| | | | | | |
|---|---|---|---|---|---|
| nd: no data *Scoring:* 0: no cytopathic effect (CPE), cells appeared to be the same as in the uninfected control 1: very small areas showed low level of CPE 2: CPE observed in small areas of the cell culture 3: stronger CPE, but not as strong as in the infected control 4: CPE is as strong as in the infected control | | | | | |

Quantitative PCR analysis revealed that Azelastine was highly effective to reduce the viral particle numbers, both in a co-administration (simulating prevention) up to >99% and up to >97% in post-infection (simulating post-exposure or therapy) administration settings (Table 2) suggesting that Azelastine can be used in both prophylactic and therapeutic settings. As expected, the co-administration is more effective, but the low viral numbers at the 25 µm Azelastine concentration demonstrates that ongoing infection can be stopped not only prevented.

**Table 2**

| | **viral particle/µL** | |
|---|---|---|
| **Azelastine concentration (µM)** | **co-administration** | **post-infection administration** |
| **0** | 99.15 | 99.15 |
| **3,125** | 83.80 | 111.00 |
| **6,25** | 15.20 | 47.05 |
| **12,5** | 7.36 | 52.00 |
| **25** | 0.20 | 2.33 |

Numbers represent median values of up to 5 replicate samples/concentration.

## Claims

1. An Azelastine compound in an antiviral effective amount for use as an antiviral substance in a pharmaceutical preparation for use in prophylactic or therapeutic treatment of a subject in need of antiviral treatment.

2. The Azelastine compound for use according to claim 1, wherein a disease condition is treated which is caused by or associated with an infection by a Coronaviridae virus, preferably selected from the group consisting of a β-coronavirus, SARS-CoV-2, MERS-CoV, SARS-CoV-1, HCoV-OC43, HCoV-HKU1, and an α-coronavirus, such as HCoV-NL63, HCoV-229E or PEDV.

3. The Azelastine compound for use according to claim 1 or 2, wherein the pharmaceutical preparation is a medicinal product or a drug product, comprising the Azelastine compound and a pharmaceutically acceptable carrier.

4. The Azelastine compound for use according to any one of claims 1 to 3, wherein the disease condition is common cold, infection of the nose, sinusitis, throat and larynx, bronchiolitis, diarrhea, rash on skin, or pneumonia, acute respiratory distress syndrome (ARDS).

5. The Azelastine compound for use according to any one of claims 1 to 4, wherein the antiviral effective amount is effective in preventing infection of susceptible cells by the virus, thereby treating the disease condition.

6. The Azelastine compound for use according to any one of claims 1 to 4, wherein the antiviral effective amount is 0.1 - 500 µg /dose.

7. The Azelastine compound for use according to any one of claims 1 to 6, wherein said pharmaceutical preparation is formulated for local administration, preferably for application to the upper and lower respiratory tract, nasal, pulmonary, intraoral, ocular, or dermal use, or for systemic administration, preferably by intravenous, intramuscular, subcutaneous, intradermal, transdermal, or oral administration.

8. The Azelastine compound for use according to any one of claims 1 to 7, wherein said pharmaceutical preparation is administered to the subject as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

9. The Azelastine compound for use according to any one of claims 1 to 8, wherein the Azelastine compound is applied into the subject's nose in an antiviral effective amount of 1-1000 µg per nostril.

10. The Azelastine compound for use according to any one of claims 1 to 9, wherein the Azelastine compound is administered as the sole antiviral substance, or wherein treatment is combined with a further treatment with one or more active substances, preferably selected from the group consisting of antiviral, anti-inflammatory and antibiotic substances.

11. The Azelastine compound for use according to any one of claims 1 to 10, wherein a subject is treated who has been infected or is at risk of being infected with said virus, preferably a human being, dog, cat, horse, camelids, cattle or pig.

12. An Azelastine compound for use as an antiviral substance in a medicinal product for treating a biological surface to prevent from virus infection and/or virus spread.

13. The Azelastine compound for use according to claim 12, wherein the biological surface is a mucosal surface which is infected or at risk of being infected with said virus.

14. The Azelastine compound for use according to claim 12 or 13, wherein said virus is a Coronaviridae virus, preferably selected from the group consisting of a β-coronavirus, such as SARS-CoV-2, MERS-CoV, SARS-CoV-1, HCoV-OC43, or HCoV-HKU1, or an α-coronavirus, such as HCoV-NL63, HCoV-229E or PEDV.

15. The Azelastine compound for use according to any one of claims 12 to 14, wherein said medicinal product is formulated for topical use, preferably for application to the upper and lower respiratory tract, nasal, pulmonary, intraoral, ocular, or dermal application.

16. The Azelastine compound for use according to any one of claims 12 to 15, wherein said medicinal product is used as a solution, dispersion, dry powder, or aerosolized liquid or powder.

17. The Azelastine compound for use according to any one of claims 12 to 16, wherein the Azelastine compound is applied in an antiviral effective amount, preferably wherein the amount is 1 ng -1000 ng / cm².

18. The use of an Azelastine compound as viral disinfectant.
